# EUROPÄISCHE PATENTSCHRIFT

(11) **EP 0 342 416 B1**
(45) Veröffentlichungstag und Bekanntmachung des Hinweises auf die Patenterteilung: **21.04.1993**
(21) Anmeldenummer: 89107757.0
(22) Anmeldetag: 28.04.1989
(51) Int. Cl.: A61K 35/38, A61K 35/78, A61K 7/26

(54) **Arzneimittel**
Medicine
Médicament

(30) Priorität: 20.05.1988 DE 3817360
(43) Veröffentlichungstag der Anmeldung: 23.11.1989
(73) Patentinhaber: Zoubek, Gerhard, D-81479 München (DE); Zoubek, Wolfgang, D-80809 München (DE); SANUM-KEHLBECK GmbH & Co. KG, D-27318 Hoya (DE)
(72) Erfinder: Zoubek, Eugen, Dr., D- (DE); Kehlbeck, Heinrich, D-2812 Hoya (DE)
(74) Vertreter: Tetzner, Volkmar, Dr.-Ing. Dr. jur.

(56) Entgegenhaltungen:
- EP-A- 0 049 379
- EP-A- 0 183 253
- List, P.H. et al, ARZNEIFORMLEHRE , 1985 , Seite 287

## Beschreibung

Die Erfindung betrifft ein Arzneimittel, eine biologische Zahncreme, ein Lyophilisat eines Organextraktes sowie ein Verfahren zur Herstellung eines Arzneimittels.

Durch die EP-A-0 183 253 ist ein Arzneimittel in Form eines aus den Peyer'schen Plaques hergestellten Organextraktes bekannt. Es läßt sich vorteilhaft zur Verbesserung des humoralen Abwehrsystems einsetzen.

Durch die EP-A-0 049 379 ist es ferner bekannt, aus den Peyer'schen Plaques einen Faktor zur Stimulation der Proliferationsrate von Leberzellen zu isolieren.

Schließlich ist es bekannt (vgl. etwa P.H.List, Arzneiformenlehre, Wissenschaftliche Verlagsgesellschaft mbH Stuttgart, 1985), zur Konservierung von Blut, Plasmafraktionen, Extrakten aus tierischem Material, Therapeutika, Grundnahrungsmitteln oder pflanzlichen Arzneizubereitungen die Lyophilisation (Gefriertrocknung) zu verwenden.

Der Erfindung liegt die Aufgabe zugrunde,
- das Arzneimittel gemäß EP-A-0 183 253 dahin weiterzuentwickeln, daß es eine verbesserte Wirksamkeit bei gleichzeitig guter Verträglichkeit erhält,
- vorteilhafte Verwendungen dieses Arzneimittels anzugeben,
- und ein geeignetes Verfahren zu seiner Herstellung zu entwickeln.

Diese Aufgabe wird - was das Arzneimittel in Form eines aus den Peyer'schen Plaques hergestellten Organextraktes anbelangt - dadurch gelöst, daß es als homöopathisches Arzneimittel ausgebildet ist, dessen Ursubstanz ein Lyophilisat des aus den Peyer'schen Plaques hergestellten Organextraktes ist.

Das Lyophilisat enthält hierbei vorteilhaft den Gesamtextrakt der Peyer'schen Plaques, und zwar vorzugsweise mit Ausnahme von durch einen Hitzeschritt ausgefällten und abgefilterten thermolabilen Proteinen.

Das erfindungsgemäße Arzneimittel weist zweckmäßig eine Verdünnung entsprechend dem deutschen homöopathischen Arzneibuch (HAB) auf.

Ein erfindungsgemäßes Arzneimittel in Form eines biologischen Geriatrikums ist durch folgende Zusammensetzung gekennzeichnet:

| | |
|---|---|
| Ovula Cheloniae | 80 bis 100 g |
| Ginseng silvestris | 3 bis 5 g |
| Extr. Fol. Hellebor niger | 2 bis 4 g |
| Germanium Citrat/Lactat (Sanumgerman ®) | 1 bis 2 g |
| Extr. Papayae | 80 bis 100 g |
| Extr. Vanill. | 40 bis 42 g |
| Caenzym Q₁₀ | 1 bis 2 g |
| Lecithin liqu. (Soja) | 18 bis 20 g |
| Rebas ® D4 | 50 bis 60 g |
| Alkohol 20 % | ad 750 g |

Soweit vorstehend und bei den im folgenden genannten Arzneimitteln und Stoffen als Bestandteil "Rebas ®" genannt ist, ist dies ein Lyophilisat des Gesamtextraktes der Peyer'schen Plaques in homöopathischer Verdünnung (beispielsweise in der homöopathischen Potenz D4).

Bei dem vorstehend genannten biologischen Geriatrikum dient der Bestandteil "Rebas ®" dazu, das sich im Alter rückbildende Immunorgan zu aktivieren.

Ein erfindungsgemäßes Arzneimittel in Form einer homöopathischen antiviralen Salbe ist durch folgende Zusammensetzung gekennzeichnet:

| | |
|---|---|
| Cantharis D2 | 3 bis 5 g |
| Rhus toxicodendron D2 | 3 bis 5 g |
| Acid nictricum D4 | 3 bis 5 g |
| Extractum Fol. Melissae | 0,2 g |
| Rebas ® D4 | 3 bis 5 g |
| Ozonid SV | 3 bis 5 g |

Eine derartige Salbe dient zur raschen Beseitigung des Herpes labialis sowie zur zusätzlichen erfolgreichen Behandlung der Gürtelrose.

Ein erfindungsgemäßes Arzneimittel in Form eines biologischen Antiallergikums ist durch folgende Zusammensetzung gekennzeichnet:

| | |
|---|---|
| Galphimia glauca D3 | 8 bis 10 g |
| Cardiospermum D3 | 8 bis 10 g |
| Apis mellifica D4 | 18 bis 20 g |
| Urtica D4 | 8 bis 10 g |
| Rebas ® | 8 bis 10 g |
| Okoubaka D3 | 8 bis 10 g |
| Acid Formicicum D12 | 8 bis 10 g |
| Histamin D30 | 8 bis 10 g |
| Glandula suprarenalis D6 | 8 bis 10 g |

Bei einem derartigen Antiallergikum dient der erfindungsgemäße Bestandteil "Rebas ®" dazu, die bei der Allergie gestörte Abwehr wiederherzustellen.

Gegenstand der Erfindung ist weiterhin eine biologische Zahncreme, die durch einen in homöopathischer Verdünnung enthaltenen Bestandteil eines aus dem Gesamtextrakt der Peyer'schen Plaques hergestellten Lyophilisats gekennzeichnet ist.

Eine derartige Zahncreme kann erfindungsgemäß folgende Zusammensetzung aufweisen:

| | |
|---|---|
| Symphytum | 8 bis 10 g |
| Rebas ® D4 | 0,5 bis 1 g |
| Rad. Ratanhia | 8 bis 10 g |
| Tct. Echinacea | 0,5 bis 1 g |
| Propolis | 0,5 bis 1 g |
| Essent. Menth. pip. | 8 bis 10 g |
| Mannit | 0,01 bis 0,03 g |
| Ozon SV (sorbinsaure Ozonitsalbe) | 3 bis 5 g |
| Dextrose (Füllmittel) | 62,97 bis 71,49 g |

Die "Rebas ®"-Komponente dieser Zahncreme dient hierbei dazu, die bakteriellen Vorgänge zu unterbinden, aus denen die für den Zahnschmelz schädlichen Säuren resultieren.

Gegenstand der Erfindung ist weiterhin ein Lyophilisat eines aus den Peyer'schen Plaques hergestellten Organextraktes zur Verwendung als Ursubstanz eines homöopathischen Arzneimittels
- zur Behandlungen von Entzündungen, insbesondere von chronischen und rezidivierenden Entzündungen, bei denen Defekte der humoralen Abwehr vorliegen,
- zur Immunmodulation der humoralen Immunität,
- zur Behandlung von Magen-Darm-Erkrankungen, wie Ulcus centriculi, Ulcus Duodeni, Colitis ulcerosa, Morbus Crohn, Diverticulitis und Pankreatitis,
- zur Behandlung von Psoriasis,
- zur unterstützenden Behandlung bei Diabetes mellitus,
- zur Behandlung von Atrophie der Magenschleimhaut, insbesondere nach Magenresektionen wegen Magenkarzinom oder Magengeschwüren, sowie
- zur Verwendung für eine der vorstehend genannten Behandlungen in Verbindung mit Thymus-Organextrakt.

Die Herstellung des erfindungsgemäßen Arzneimittels erfolgt zweckmäßig wie folgt:
Für die Herstellung des Gesamtextraktes der Peyer'schen Plaques wird dir Dünndarm von jungen, organisch gesunden, biologisch aufgezogenen Kälbern verwendet, die während der Aufzucht und der Schlachtung gesundheitlich überprüft wurden. Unmittelbar nach der Schlachtung werden die Organteile von erfahrenen Tierärzten entnommen, sorgfältig gereinigt und in flüssigem Stickstoff schockgefroren. Später werden die Organteile aufgetaut und in steriler 0,9 %iger Natriumchloridlösung gespült, bis sie absolut sauber sind. Evtl. anhaftendes Binde- und Fettgewebe wird entfernt. Die Organteile werden in einem Mixer 30 Sekunden homogenisiert. Von dem entstandenen Homogenisat wird ein Teil in 2,5 Teilen gereinigtem Wasser (DAB 9), welches auf 4 - 8° C gekühlt wurde, suspendiert und bei 4 - 8° C 60 Minuten unter Rühren extrahiert.

Danach wird die Lösung durch Verbandmull aus Baumwolle (DAB 9) dekantiert und mittels einer Durchlaufzentrifuge mit 17.000 g bei der Durchflußrate von 5 l/h zentrifugiert. Der Überstand wird durch einen Membranfilter aus Glasfasermaterial vorfiltriert und einer Sterilfiltration mit einem Polyvinylidenfluorid-Membranfilter (0,2 µm Porengröße) unterworfen. Die sterilfiltrierte Lösung wird dann 30 Minuten bei 100° C gekocht; die ausgefallenen Proteine werden erneut abfiltriert. Dann wird dem Organextrakt das Wasser durch Lyophilisation entzogen. Letztere erfolgt nach Einfrieren des Extraktes bei - 50° C unter einem Vakuum von max. 0,4 mbar (entsprechend einer Eistemperatur von - 30° C) bei gleichzeitiger Heizung der Trocknungskammer auf 28 - 32° C).

In umfangreichen Testreihen konnten bei der Anwendung des Lyophilisats in Form von Kapseln, Salben, Zäpfchen, Injektionen, Tropfen und Tabletten überzeugende Ergebnisse bezüglich Stabilität oder Wirksamkeit gefunden werden.

Eine pharmakologisch-toxikologische Prüfung des Lyophilisats eines Gesamtextraktes aus den Peyer'schen Plaques ergab, daß die Testsubstanz nach einmaliger intramuskulärer Applikation an der Ratte keine klinisch-toxikologischen Symptome ergab.

Die mit dem erfindungsgemäßen homöopathischen Arzneimittel überraschenden gewonnenen Therapieergebnisse seien an folgenden Beispielen erläutert:
1. Ein 74-jähriger Patient wurde seit eineinhalb Jahren vor allem nachts von einer chronischen Laryngitis mit Hustenreiz und Kitzelgefühl im Kehlkopf gequält. Morgens erfolgte Auswurf von eitrigem Schleim, zeitweise Heiserkeit. Es bestanden entzündete Narben nach einer Abszeßtonsillektomie (vor 45 Jahren). Nach acht Injektionen mit Rebas® D12, gemischt mit dem Lymphmittel Injectio lymphatica in die Tonsillektomienarben, verschwanden alle Krankheitserscheinungen. Das Lymphmittel allein hatte vorher keinerlei Wirkung gezeigt.
2. Rheumatoide Arthritis im rechten Hand- und rechten Ellbogengelenk sowie erhebliche Coxarthrose links bei einem 40-jährigen Patienten seit fünf Jahren bestehend; CPR-Test pos. +++, BKS 24/55. Auch eine stationäre Behandlung in einer Münchener Universitätsklinik erbrachte keinerlei Erfolg. Die Schmerzen steigerten sich im linken Hüftgelenk bis zur Unerträglichkeit, so daß sich der Patient während und nach dem stationären Aufenthalt nur mit Hilfe von Gehhilfen bewegen konnte. Eine deutliche Bewegungseinschränkung mit aufgehobener Rotation wurde durch eine orthopädische Untersuchung bestätigt. Von orthopädischer Seite wurde auf längere Sicht eine operative Therapie der Coxarthrose mit TEP vorgeschlagen. Außerdem bestand eine Allergie. Eine biologische Behandlung erbrachte nur geringe Erfolge. Erst die Behandlung mit dem erfindungsgemäßen Extrakt ergab einen signifikanten Erfolg. Der Patient kann wieder ohne Gehhilfen gehen und ausgedehnte Wanderungen durchführen. Es besteht keine Bewegungseinschränkung im linken Hüftgelenk, ebenso sind die Schmerzen beseitigt.
3. Bei einem 65-jährigen Patienten trat ein akuter Anfall von Allergie auf mit ausgedehnten Schwellungen und Rötungen am gesamten Körper. Vorher hatte er niemals an allergischen Erscheinungen gelitten. Mit Rebas® D12 und später Rebas® D4 subcutan in die Akupunkturpunkte verschwanden sofort sämtliche allergischen Erscheinungen.
4. Bei einem 12-jährigen Mädchen war die humorale Immunabwehr stark herabgesetzt. Es folgte Erkältung auf Erkältung - oft mit hochfieberhaften Schüben, so daß vom Hausarzt öfters Antibiotika eingesetzt wurden. Die Tonsillen waren stark vergrößert und links mit Eiterpfröpfen versehen. Im Blutbild wurde eine Leukocytose bei verminderter Monocytenzahl festgestellt. Die subakuten Entzündungen wurden oral behandelt, daneben 2mal täglich 1 g Vitamin C. Die Ernährung wurde auf eine biologische Vollkornkost umgestellt. 2 Wochen später wurde mit einer Rebas®-Kur begonnen, wobei zweimal täglich eine Kapsel D4 und einmal täglich ein Zäpfchen Rebas D3 verabreicht wurde. Schon nach wenigen Wochen kam es zum Abklingen aller Entzündungserscheinungen und zur Normalisierung des Blutbildes. Es traten keine Infekte mehr auf.

## Patentansprüche

1. Arzneimittel in Form eines aus den Peyer'schen Plaques hergestellten Organextraktes, dadurch gekennzeichnet, daß es als homöopathisches Arzneimittel ausgebildet ist, dessen Ursubstanz ein Lyophilisat des aus den Peyer'schen Plaques hergestellten Organextraktes ist.

2. Arzneimittel nach Anspruch 1, dadurch gekennzeichnet, daß das Lyophilisat den Gesamtextrakt der Peyer'schen Plaques enthält.

3. Arzneimittel nach Anspruch 1, dadurch gekennzeichnet, daß das Lyophilisat den Gesamtextrakt der Peyer'schen Plaques mit Ausnahme von durch einen Hitzeschritt ausgefällten und abgefilterten thermolabilen Proteinen enthält.

4. Arzneimittel nach Anspruch 1, gekennzeichnet durch eine Verdünnung entsprechend dem deutschen Homöopathischen Arzneibuch (HAB).

5. Arzneimittel nach Anspruch 4 in Form eines biologischen Geriatrikum, gekennzeichnet durch folgende Zusammensetzung:
| | |
|---|---|
| Ovula Cheloniae | 80 bis 100 g |
| Ginseng silvestris | 3 bis 5 g |
| Extr.Fol. Hellebor niger | 2 bis 4 g |
| Germanium Citrat/Lactat (Sanumgerman®) | 1 bis 2 g |
| Extr. Papayae | 80 bis 100 g |
| Extr. Vanill. | 40 bis 42 g |
| Caenzym Q₁₀ | 1 bis 2 g |
| Lecithin liqu. (Soja) | 18 bis 20 g |
| Rebas® D4 | 50 bis 60 g |
| Alkohol 20 % | ad 750 g |

6. Arzneimittel nach Anspruch 4 in Form einer homöopathischen antiviralen Salbe, gekennzeichnet durch folgende Zusammensetzung:
| | |
|---|---|
| Cantharis D2 | 3 bis 5 g |
| Rhus toxicodendron D2 | 3 bis 5 g |
| Acid nictricum D4 | 3 bis 5 g |
| Extractum Fol. Melissae | 0,2 g |
| Rebas® D4 | 3 bis 5 g |
| Ozonid SV | 3 bis 5 g |

7. Arzneimittel nach Anspruch 4 in Form eines biologischen Antiallergikums, gekennzeichnet durch folgende Zusammensetzung:
| | |
|---|---|
| Galphimia glauca D3 | 8 bis 10 g |
| Cardiospermum D3 | 8 bis 10 g |
| Apis mellifica D4 | 18 bis 20 g |
| Urtica D4 | 8 bis 10 g |
| Rebas® D3 | 8 bis 10 g |
| Okoubaka D3 | 8 bis 10 g |
| Acid formicicum D12 | 8 bis 10 g |
| Histamin D30 | 8 bis 10 g |
| Glandula suprarenalis D6 | 8 bis 10 g |

8. Biologische Zahncreme, gekennzeichnet durch einen in homöopathischer Verdünnung enthaltenen Bestandteil eines aus dem Gesamtextrakt der Peyer'schen Plaques hergestellten Lyophilisats.

9. Zahncreme nach Anspruch 8, gekennzeichnet durch folgende Zusammensetzung:
| | |
|---|---|
| Symphytum | 8 bis 10 g |
| Rebas® D4 | 0,5 bis 1 g |
| Rad. Ratanhia | 8 bis 10 g |
| Tct. Echinacea | 0,5 bis 1 g |
| Propolis | 0,5 bis 1 g |
| Essent.Menth.pip. | 8 bis 10 g |
| Mannit | 0,01 bis 0,03 g |
| Ozon SV (sorbinsaure Ozonitsalbe) | 3 bis 5 g |
| Dextrose (Füllmittel) | 62,97 bis 71,49 g |

10. Lyophilisat eines aus den Peyer'schen Plaques hergestellten Organextraktes zur Verwendung als Ursubstanz eines homöopathischen Arzneimittels zur Behandlungen von Entzündungen, insbesondere von chronischen und rezidivierenden Entzündungen, bei denen Defekte der humoralen Abwehr vorliegen.

11. Lyophilisat eines aus den Peyer'schen Plaques hergestellten Organextraktes zur Verwendung als Ursubstanz eines homöopathischen Arzneimittels zur Immunmodulation der humoralen Immunität.

12. Lyophilisat eines aus den Peyer'schen Plaques hergestellten Organextraktes zur Verwendung als Ursubstanz eines homöopathischen Arzneimittels zur Behandlung von Magen-Darm-Erkrankungen, wie Ulcus centriculi, Ulcus Duodeni, Colitis ulcerosa, Morbus Crohn, Diverticulitis und Pankreatitis.

13. Lyophilisat eines aus den Peyer'schen Plaques hergestellten Organextraktes zur Verwendung als Ursubstanz eines homöopathischen Arzneimittels zur Behandlung von Psoriasis.

14. Lyophilisat eines aus den Peyer'schen Plaques hergestellten Organextraktes zur Verwendung als Ursubstanz eines homöopathischen Arzneimittels zur unterstützenden Behandlung bei Diabetes mellitus.

15. Lyophilisat eines aus den Peyer'schen Plaques hergestellten Organextraktes zur Verwendung als Ursubstanz eines homöopathischen Arzneimittels zur Behandlung von Atrophie der Magenschleimhaut, insbesondere nach Magenresektionen wegen Magenkarzinom oder Magengeschwüren.

16. Lyophilisat eines aus den Peyer'schen Plaques hergestellten Organextraktes zur Verwendung als Ursubstanz eines homöopathischen Arzneimittels zur Verwendung für eine der in den Ansprüchen 10 bis 15 genannten Behandlungen in Verbindung mit Thymus-Organextrakt.

17. Verfahren zur Herstellung eines Arzneimittels gemäß Anspruch 1, dadurch gekennzeichnet, daß der Organextrakt der Peyer'schen Plaques aus dem Dünndarm von jungen, biologisch aufgezogenen Kälbern hergestellt wird.

## Claims

1. Medicament in the form of an organ extract produced from Peyer's patches, characterised in that it is formed as a homoeopathic medicament, the primary substance of which is a lyophilisate produced from Peyer's patches.

2. Medicament as claimed in claim 1, characterised in that the lyophilisate contains the total extract of Peyer's patches.

3. Medicament as claimed in claim 1, characterised in that the lyophilisate contains the total extract of Peyer's patches with the exception of thermolabile proteins which are precipitated by a heating step and filtered off.

4. Medicament as claimed in claim 1, characterised by dilution in accordance with the German Homöopathisches Arzneibuch (HAB) (*Book of Homoeopathic Medicine*).

5. Medicament as claimed in claim 4 in the form of a biological geriatric medicine, characterised by the following composition:
| | |
|---|---|
| Ovula Cheloniae | 80 to 100 g |
| Ginseng silvestris | 3 to 5 g |
| Extr. Fol. Hellebor niger | 2 to 4 g |
| Germanium citrate/lactate (Sanumgerman®) | 1 to 2 g |
| Extr. Papayae | 80 to 100 g |
| Extr. Vanill. | 40 to 42 g |
| Caenzym Q₁₀ | 1 to 2 g |
| Lecithin liqu. (Soya) | 18 to 20 g |
| Rebas® D4 | 50 to 60 g |
| Alcohol 20% | ad 750 g |

6. Medicament as claimed in claim 4 in the form of a homoeopathic anti-viral ointment, characterised by the following composition:
| | |
|---|---|
| Cantharis D2 | 3 to 5 g |
| Rhus toxicodendron D2 | 3 to 5 g |
| Acid nictricum D4 | 3 to 5 g |
| Extractum Fol. Melissae | 0.2 g |
| Rebas® D4 | 3 to 5 g |
| Ozonide SV | 3 to 5 g |

7. Medicament as claimed in claim 4 in the form of a biological anti-allergic medicine, characterised by the following composition:
| | |
|---|---|
| Galphimia glauca D3 | 8 to 10 g |
| Cardiospermum D3 | 8 to 10 g |
| Apis mellifica D4 | 18 to 20 g |
| Urtica D4 | 8 to 10 g |
| Rebas® D3 | 8 to 10 g |
| Okoubaka D3 | 8 to 10 g |
| Acid formicicum D12 | 8 to 10 g |
| Histamine D30 | 8 to 10 g |
| Glandula suprarenalis D6 | 8 to 10 g |

8. Biological toothpaste, characterised by a constituent in homoeopathic dilution of a lyophilisate produced from the total extract of Peyer's patches.

9. Toothpaste as claimed in claim 8, characterised by the following composition:
| | |
|---|---|
| Symphytum | 8 to 10 g |
| Rebas® D4 | 0.5 to 1 g |
| Rad. Ratanhia | 8 to 10 g |
| Tct. Echinacea | 0.5 to 1 g |
| Propolis | 0.5 to 1 g |
| Essent. Menth. pip. | 8 to 10 g |
| Mannite | 0.01 to 0.03 g |
| Ozone SV (sorbic acid ozonite ointment) | 3 to 5 g |
| Dextrose (filler) | 62.97 to 71.49 g |

10. Lyophilisate of an organ extract produced from Peyer's patches for use as the primary substance of a homoeopathic medicament for the treatment of inflammations, particularly chronic and recurrent inflammations, in which defects of the humoral defences occur.

11. Lyophilisate of an organ extract produced from Peyer's patches for use as the primary substance of a homoeopathic medicament for the immunomodulation of the humoral immunity.

12. Lyophilisate of an organ extract produced from Peyer's patches for use as the primary substance of a homoeopathic medicament for the treatment of gastrointestinal illnesses, such as ulcus ventriculi, ulcus duodeni, colitis ulcerosa, morbus Crohn, diverticulitis and pancreatitis.

13. Lyophilisate of an organ extract produced from Peyer's patches for use as the primary substance of a homoeopathic medicament for the treatment of psoriasis.

14. Lyophilisate of an organ extract produced from Peyer's patches for use as the primary substance of a homoeopathic medicament for supportive treatment in the case of diabetes mellitus.

15. Lyophilisate of an organ extract produced from Peyer's patches for use as the primary substance of a homoeopathic medicament for the treatment of atrophy of the gastric mucosa, particularly after stomach resections due to carcinoma of the stomach or gastric ulcers.

16. Lyophilisate of an organ extract produced from Peyer's patches for use as the primary substance of a homoeopathic medicament for one of the treatments referred to in claims 10 to 15 in combination with thymus organ extract.

17. Method of producing a medicament as claimed in claim 1, characterised in that the organ extract of Peyer's patches is produced from the small intestine of young biologically reared calves.

## Revendications

1. Médicament sous forme d'un extrait d'organe préparé à partir des plaques de Peyer, caractérisé en ce qu'il est constitué d'un médicament homéopathique dont la substance de base est un lyophilisat de l'extrait d'organe préparé à partir des plaques de Peyer.

2. Médicament selon la revendication 1, caractérisé en ce que le lyophilisat contient l'extrait total des plaques de Peyer.

3. Médicament selon la revendication 1, caractérisé en ce que le lyophilisat contient l'extrait total des plaques de Peyer à l'exclusion des protéines thermolabiles précipitées par une étape thermique et séparées par filtration.

4. Médicament selon la revendication 1, caractérisé par une dilution correspondant au répertoire homéopathique allemand (HAB).

5. Médicament selon la revendication 4 sous forme d'une préparation biologique à usage gériatrique, caractérisé par la composition suivante :
| | |
|---|---|
| Ovula Cheloniae | 80 à 100 g |
| Ginseng silvestris | 3 à 5 g |
| Extr. Fcl. Hellebor niger | 2 à 4 g |
| Citrate/lactate de germanium (Sanumgerman ®) | 1 à 2 g |
| Extr. Papayae | 80 à 100 g |
| Extr. Vanill. | 40 à 42 g |
| Coenzyme, Q₁₀ | 1 à 2 g |
| Lécithine liqu. (Soja) | 18 à 20 g |
| Rebas ® D4 | 50 à 60 g |
| Alcool à 20 % | complément à 750 g |

6. Médicament selon la revendication 4 sous forme d'une pommade antivirale homéopathique caractérisé par la composition suivante :
| | |
|---|---|
| Cantharis D2 | 3 à 5 g |
| Rhus toxicodendron D2 | 3 à 5 g |
| Acid nictricum D4 | 3 à 5 g |
| Extractum Fol. Melissae | 0,2 g |
| Rebas ® D4 | 3 à 5 g |
| Ozonid SV | 3 à 5 g |

7. Médicament selon la revendiation 4 sous forme d'un antiallergique biologique, caractérisé par la composition suivante :
| | |
|---|---|
| Galphimia glauca D3 | 8 à 10 g |
| Cardiospermum D3 | 8 à 10 g |
| Apis mellifica D4 | 18 à 20 g |
| Urtica D4 | 8 à 10 g |
| Rebas ® D3 | 8 à 10 g |
| Okoubaka D3 | 8 à 10 g |
| Acid formicicum D12 | 8 à 10 g |
| Histamine D30 | 8 à 10 g |
| Glandula suprarenalis D6 | 8 à 10 g |

8. Pâte dentifrice biologique, caractérisée par un constituant, contenu en dilution homéopathique, d'un lyophilisat préparé à partir de l'extrait total des plaques de Peyer.

9. Pâte dentifrice selon la revendication 8, caractérisée par la composition suivante :
| | |
|---|---|
| Symphytum | 8 à 10 g |
| Rebas ® D4 | 0,5 à 1 g |
| Rad. Ratanhia | 8 à 10 g |
| Tct. Echinacea | 0,5 à 1 g |
| Propolis | 0,5 à 1 g |
| Essent. Menth. pip. | 8 à 10 g |
| Mannitol | 0,01 à 0,03 g |
| Ozon SV (pommade d'ozonite à l'acide sorbique) | 3 à 5 g |
| Dextrose (excipient) | 62,97 à 71,49 g |

10. Lyophilisat d'un extrait d'organe préparé à partir des plaques de Peyer pour l'utilisation comme substance de base d'un médicament homéopathique destiné au traitement d'inflammations, notamment d'inflammations chroniques et récidivantes, dans lesquelles est impliqué un déficit du système de défense humoral.

11. Lyophilisat d'un extrait d'organe préparé à partir des plaques de Peyer pour l'utilisation comme substance de base d'un médicament homéopathique destiné à la modulation du système immunitaire humoral.

12. Lyophilisat d'un extrait d'organe préparé à partir des plaques des Peyer pour l'utilisation comme substance de base d'un médicament homéopathique destiné au traitement de maladies gastrointestinales telles que Ulcus ventriculi, Ulcus duodeni, Colitis ulcerosa, la maladie de Crohn la diverticulite et la pancréatite.

13. Lyophilisat d'un extrait d'organe préparé à partir des plaques de Peyer pour l'utilisation comme substance de base d'un médicament homéopathique pour le traitement du psoriasis.

14. Lyophilisat d'un extrait d'organe préparé à partir des plaques de Peyer pour l'utilisation comme substance de base d'un médicament homéopathique destiné au traitement d'appoint du diabète sucré.

15. Lyophilisat d'un extrait d'organe préparé à partir des plaques de Peyer pour l'utilisation comme substance de base d'un médicament homéopathique destiné au traitement de l'atrophie de la muqueuse gastrique, notamment après des résections de l'estomac en raison d'un carcinome ou d'un ulcère de l'estomac.

16. Lyophilisat d'un extrait d'organe préparé à partir des plaques de Peyer pour l'utilisation comme substance de base d'un médicament homéopathique destiné à être utilisé pour l'un des traitements cités dans les revendications 10 à 15 conjointement avec un extrait de thymus.

17. Procédé de préparation d'un médicament selon la revendication 1, caractérisé en ce que l'extrait d'organe des plaques de Peyer est préparé à partir de l'intestin grêle de jeunes veaux élevés biologiquement.
